# EUROPEAN PATENT APPLICATION

(11) **EP 0 745 349 A1**
(43) Date of publication of application: **04.12.1996**
(21) Application number: 96201489.0
(22) Date of filing: 29.05.1996
(51) Int. Cl.: A61B 5/107, A43D 1/02

(54) **Device for producing a print form of i. e. the sole of a persons foot**

(30) Priority: 29.05.1995 NL 1000444
(71) Applicant: van der Meulen, Dirk, 9244 AJ Beetsterzwaag (NL); van der Wijk, Jacobus, 9201 JV Drachten (NL); oim Holding B.V., 9751 NN Haren (NL)
(72) Inventor: Van der Meulen, Dirk, 9244 AJ Beetsterzwaag (NL)
(74) Representative: 't Jong, Bastiaan Jacobus

(57) **Abstract**

A device for making an imprint the device comprises an at least partially deformable closed holder (2) in which is arranged a quantity of substantially incompressible and mutually displaceable granular material (16) and means (6) for clamping together the granular material for fixation purposes. A number of adjacent sides of the holder can be formed by an elastic membrane (15). The holder can be a bag formed from the elastic membrane.

## Description

The invention relates to a device for making an imprint.

Such a device is known from the German Auslegeschrift 1 170 114. This known device comprises a holder in which is arranged a quantity of substantially incompressible and mutually displaceable granular material. Stretched over this granular material on the upper part of the holder is an elastic membrane. The holder can be connected to a vacuum source. When the pressure in the holder is the same as or only a little lower than the outside air pressure, the granules in the form of balls can move easily over each other so that a shape can be pressed into the granules via the membrane. By then evacuating the holder the granules are clamped together and fixed by the elastic membrane pressed thereagainst, whereby the impressed form is retained. The thus obtained mould imprint can for instance be used to make a duplicate mould. An example of an application is the manufacture of arch supports or special footwear. Using the obtained imprint an accurate copy of the foot of a person can be obtained, so that an arch support or the footwear can be adapted precisely to the foot of this person.

The invention has for its object to further improve this known device.

In the device according to the invention this is achieved in that the holder is embodied such that a number of adjacent sides thereof are formed by an elastic membrane. In the not yet fixed or not yet completely fixed situation of the granular material, the granular material can be laid accurately against the form with the elastic membrane therebetween, in that it can be pressed from the sides or optionally the underside likewise formed by the elastic membrane. Once the granular material thus lies accurately via the elastic membrane against the form of which an imprint must be made, the material is clamped together for fixation, for instance by evacuating the holder, whereby a very accurate imprint of a very complicated shape can be obtained. Instead of by evacuating the holder, clamping together of the granular material can also be obtained by applying a magnetic field when this granular material consists of for instance steel balls.

A very suitable embodiment of the device according to the invention is characterized in claim 2. The bag filled with granular material can be manipulated very well and can be pressed firmly on all sides round the original form.

The means for clamping the granular material for fixation preferably take the form of a vacuum source and a connecting member therefor to the holder. The accessibility of the sides or the bottom of the holder is not hereby impeded by these means for clamping together the granular material. As the vacuum gradually becomes stronger the granular material can remain pressed round the form of which the imprint must be made, so that possible displacements resulting from the occurring forces are compensated.

The step of claim 4 is preferably applied. The latex has very great stretch, so that even in the case of a very highly profiled form a good contact of this membrane against the original form can be obtained without folds.

The invention also relates to and provides a device for making imprints of the foot sole of a person. This device comprises two separate, adjacently disposable holders with the above described features. The relevant person stands with each foot on one holder and assumes the most relaxed posture for this person. Herein a slight vacuum can for instance be produced in the holders to obtain sufficient support.

When the person has found the most comfortable posture, wherein his muscles are relatively relaxed, a further vacuum is produced in the holders, wherein the granular material is pressed round the feet by another person. When the form is fixed the person can take his feet out of the holders and two imprints are obtained which, when arch supports and/or special footwear are made therefrom, ensure this person a comfortable posture during walking and standing.

The invention will be further elucidated in the following description with reference to the annexed drawings.
- Figure 1: shows a perspective view of a device according to a preferred embodiment of the invention.

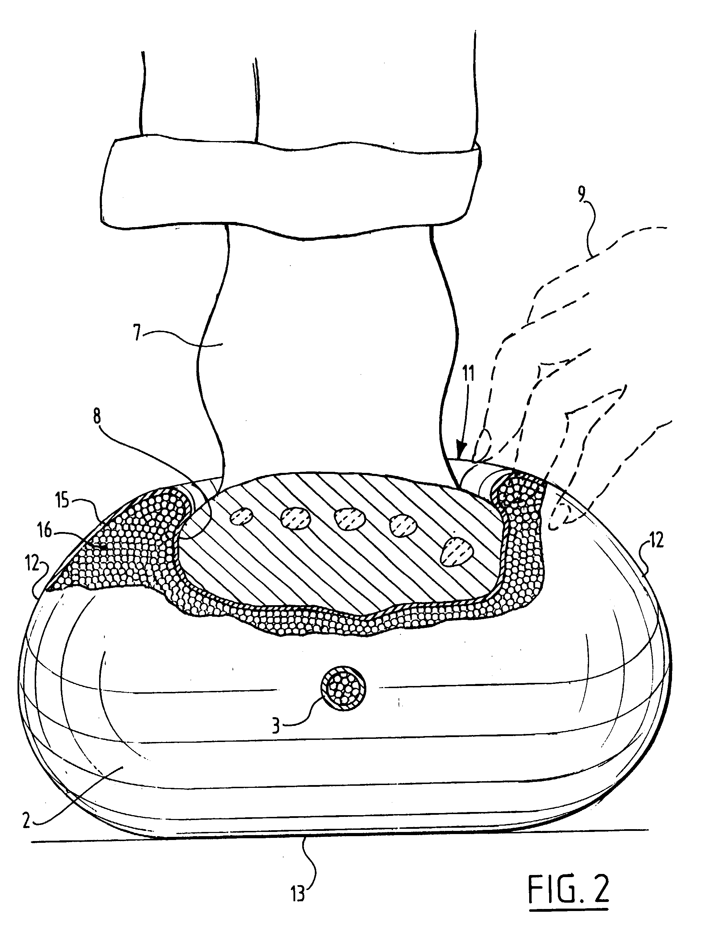

- Figure 2: shows a partly sectional front view of a part of the device during use thereof.

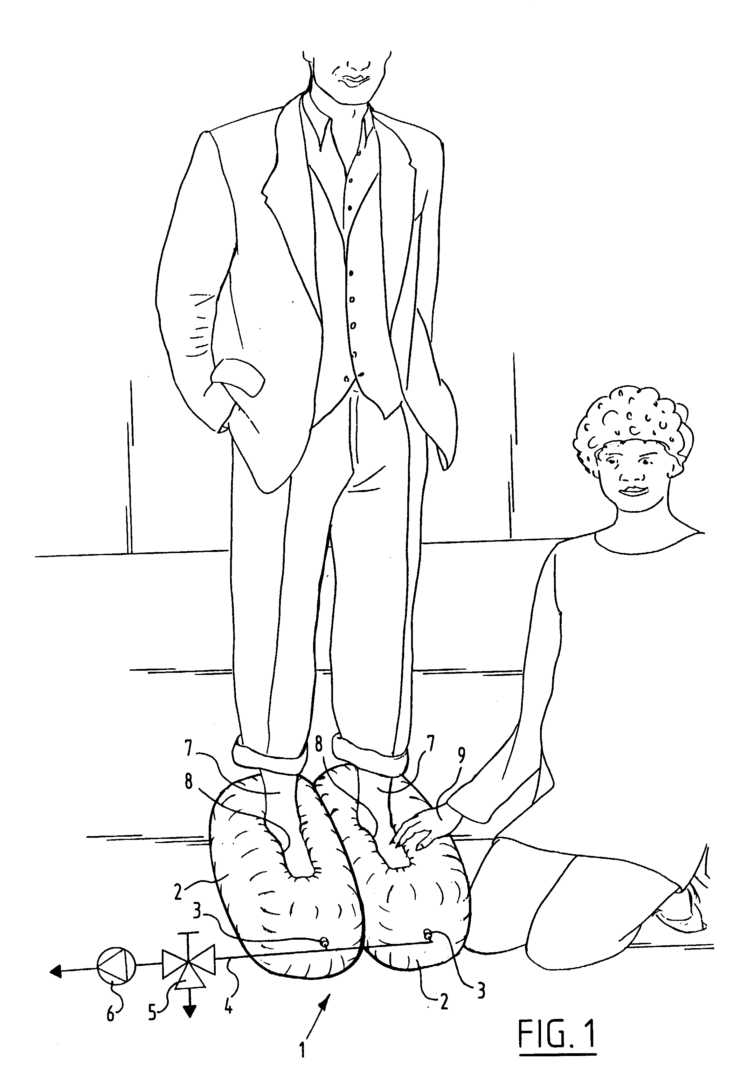

As shown in figure 1, the device 1 according to the invention comprises two holders 2 which in this embodiment are formed by a bag formed from an elastic membrane 15. Each holder 2 is filled with granular material 16 which is substantially incompressible and mutually displaceable. The granular material can suitably consist for instance of steel balls, which can slide easily along each other whereby they can properly follow a form pressed into the holder.

Connected to holders 2 are vacuum connections 3 which are connected via a schematically shown vacuum conduit 4 and valve 5 to a vacuum source embodied as a vacuum pump 6. By means of this vacuum source a vacuum can be produced in the interior of holders 2. When this takes place the membrane is pressed so firmly onto the granular material by the outside air pressure that it is clamped together and fixation of the impressed form is obtained.

In the example of an application shown in figure 1 it is the object to make an imprint 8 of the feet of a person 7, in particular of the foot soles, in order to enable manufacture of arch supports or adapted footwear with the obtained imprint 8.

For this purpose two of the described holders 2 are disposed adjacently of each other and one foot 7 of the person is placed on each of these holders. In the shown embodiment a slight vacuum can be created beforehand in the holders so that a stable entity is obtained. Due to the good mobility thereof the granular material fits closely round the feet 7 via the elastic membrane, preferably consisting of latex rubber.

Because the holder is in the form of a bag in the shown embodiment, the sides 12 of holder 2 adjacent to the top part 11 are formed by the elastic membrane 15. A person making the imprint can hereby press the granular material with his hand 9 from the sides 12 against the foot 7, so that the sides of the foot are also represented accurately in the imprint. It is even possible to press the granular material 16 in upward direction via the underside 13 in order to ensure that the material on the underside also comes into well-distributed contact with the foot. Once the granular material 16 has thus been modelled round foot 7, a vacuum is produced in holders 2, whereby granules 16 are clamped together in the above described manner such that a rigid whole is formed. After removal of the feet 7 an imprint 8 remains which corresponds precisely with the external shape of the foot 7. This shape can then be further used to manufacture the arch supports or special footwear.

The invention is not limited to the shown embodiment for manufacturing an imprint of the feet of a person. With a device according to the invention, which in its simplest form consists of only one holder 2, an imprint can be obtained of any original form. The imprint can then for instance be used as mould for making a copy. Because the granular material can be pressed from the sides a good imprint can be obtained even in the case of complex forms.

## Claims

1. Device for making an imprint, comprising an at least partially deformable closed holder in which is arranged a quantity of substantially incompressible and mutually displaceable granular material and means for clamping together said granular material for fixation purposes, wherein a number of adjacent sides of said holder are formed by an elastic membrane.

2. Device as claimed in claim 1, wherein the holder is a bag formed from the elastic membrane.

3. Device as claimed in either of the foregoing claims, wherein the means for clamping together the granular material for fixation purposes comprise a vacuum source and a connecting member therefor to the holder.

4. Device as claimed in any of the foregoing claims, wherein the elastic membrane is a latex membrane.

5. Device for making imprints of the foot soles of a person, comprising two separate, adjacently disposable devices as claimed in any of the foregoing claims, wherein the connecting members of both devices are connected to each other and to a vacuum source.
